# EUROPEAN PATENT SPECIFICATION

(11) **EP 0 394 906 B1**
(45) Date of publication and mention of the grant of the patent: **20.12.1995**
(21) Application number: 90107636.4
(22) Date of filing: 23.04.1990
(51) Int. Cl.: C09K 19/30, C07C 69/00, C07C 255/00

(54) **Liquid crystalline composition**
Flüssigkristallzusammensetzung
Composition liquide cristalline

(30) Priority: 26.04.1989 GB 8909489
(43) Date of publication of application: 31.10.1990
(73) Proprietor: MERCK PATENT GmbH, D-64271 Darmstadt (DE)
(72) Inventor: Coates, David, Dr., Wimborne, Dorset BH21 3SW (GB); Sage, Ian Charles, Dr., Broadstone, Dorset BH18 8EG (GB); Greenfield, Simon, Dr., Creekmoor, Poole BH17 7YA (GB); Smith, Graham, Poole, Dorset BH17 8AX (GB); Chambers, Michael, Poole, Dorset (GB); Kurmeier, Hans-Adolf, Dr., D-6104 Seeheim-Jugenheim (DE); Dorsch, Dieter, Dr., D-6100 Darmstadt (DE)

(56) References cited:
- EP-A- 0 019 665
- EP-A- 0 023 728
- EP-A- 0 236 215
- WO-A-88/09360
- GB-A- 2 197 868
- US-A- 4 113 647

## Description

The invention relates to liquid crystalline compositions for the use in a twisted nematic cell being an admixture of a group (A) with one or more components exhibiting a positive dielectric anisotropy (Δε) and a group (B) with one or more components exhibiting a neutral dielectric anisotropy characterized in that
(a) at least one component of group (A) is a compound of the formula I 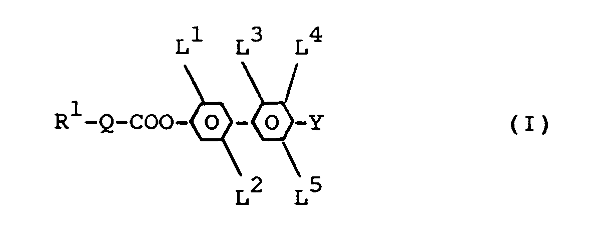 wherein
   - R¹: is an alkyl or alkenyl residue each with up to 16 C atoms, wherein one or two non-adjacent CH₂-groups of these residues may be replaced by -O-, -S-, -CO-O-, or -O-CO-,
   - Y: is CN, OCF₃, OCHF₂ or CF₃
   - Q: is a group of the formula 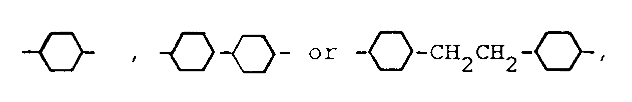 and one of the lateral ligands L¹ through L⁴ is F, the others are H, and
   - L⁵: is F or H

The invention was based on the object of discovering that new liquid crystalline composition with advantageous values for optical and dielectric anisotropy for the use in twisted nematic cells can be obtained by admixing a group (A) with one or more components exhibiting a positive dielectric anisotropy and a group (B) with one or more components exhibiting a neutral dielectric anisotropy wherein at least one component is a compound of the formulae I (group A). The compounds of the formula I are embraced by the broad general formula of EP-0 019 665 but neither compounds with laterally fluorine substituents are disclosed there nor is described how to prepare them.

The British patent application GB 21 19 868 describes cyclohexylmethylether of 2'-fluoro-, 2-fluoro- or 2,2'-difluoro-4'-cyanobiphenyl as being useful for liquid crystalline mixtures with S_{c} phases.

US-A-4 113 647 discloses a liquid crystal material comprising a trans-4-alkylcyclohexane-1-carboxylic acid ester (e.g. 4-cyanobiphenyl-4'-yl cyclohexane-carboxylate without lateral fluoro substituents)
It has now been found that fluorinated compounds of formula I are highly suitable as components of liquid crystalline compositions for the use in twisted nematic cells. In particular, they have especially advantageous values of optical and dielectric anisotropy. It is also possible to obtain stable liquid crystalline phases with a broad nematic mesophase range and a comparatively low viscosity with the aid of these compounds.

Depending on the choice of the substituents, the compounds of the formula I can be used as the base materials from which liquid crystal media are predominantly composed; however, it is also possible for compounds of the formula I to be added to liquid crystal base materials of other classes of compounds, for example in order to influence the dielectric and/or optical anisotropy and/or the viscosity and/or the mesophase range of such a dielectric.

The compounds of the formula I are colourless in the pure state and are liquid crystalline in a temperature range which is favourably placed for electrooptical use. They are very stable towards chemicals, heat and light.

The invention thus relates to a liquid crystalline composition for the use in a twisted nematic cell being an admixture of a group (A) with one or more components exhibiting a positive dielectric anisotropy (Δε) and a group (B) with one or more components exhibiting a neutral dielectric anisotropy characterized in that
at least one component of group (A) is a compound of the formula I or
In particular to liquid crystalline compositions wherein the dielectric anisotropy (Δε) of the group A is greater than +6 or wherein
the dielectric anisotropy (Δε) of the group B is -4 to +3.

Furthermore, the invention relates to a twisted nematic cell containing a liquid crystalline composition according to the invention in particular to a cell in which the twist angle is between 160 and 300 °.

Some of the compounds of the formula I are known, but some are still novel.

Therefore, the invention relates to the novel compounds of the formula I.

Above and below, R¹, Y, Q, L¹ through L⁵ have the meaning given unless expressly indicated otherwise.

The biphenyl esters of the formula I include mono-and difluorinated compounds with three or four rings of the formulae I1 to I3 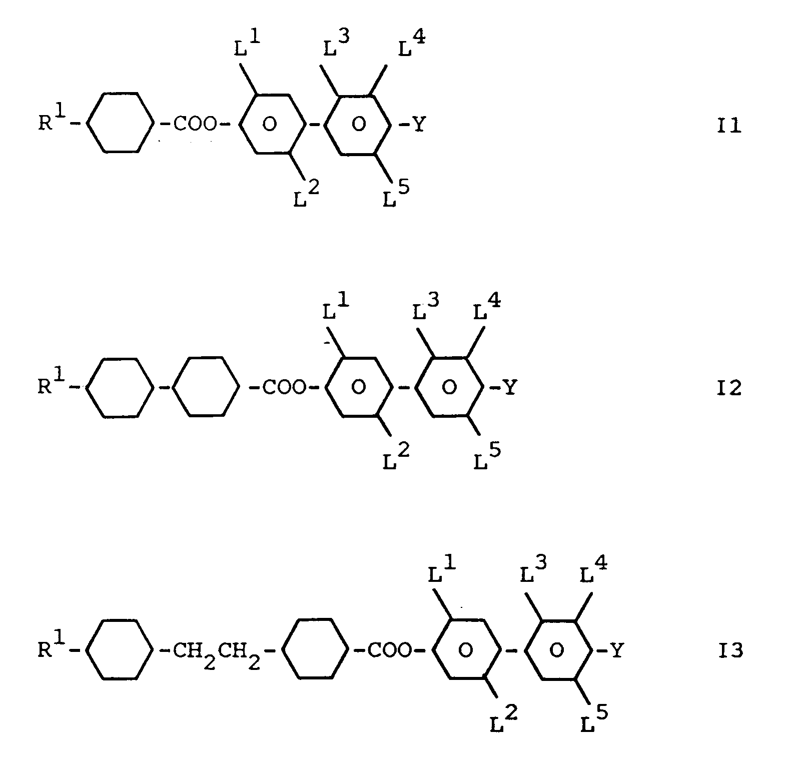
The fluorinated biphenyl unit 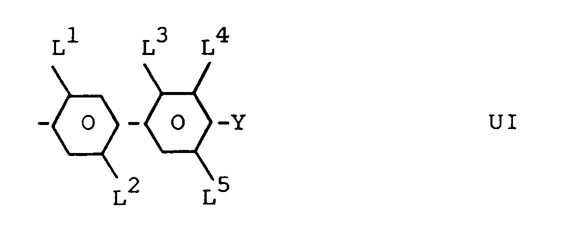
of the compounds of the formula I includes the structure elements of UI1 to UI8 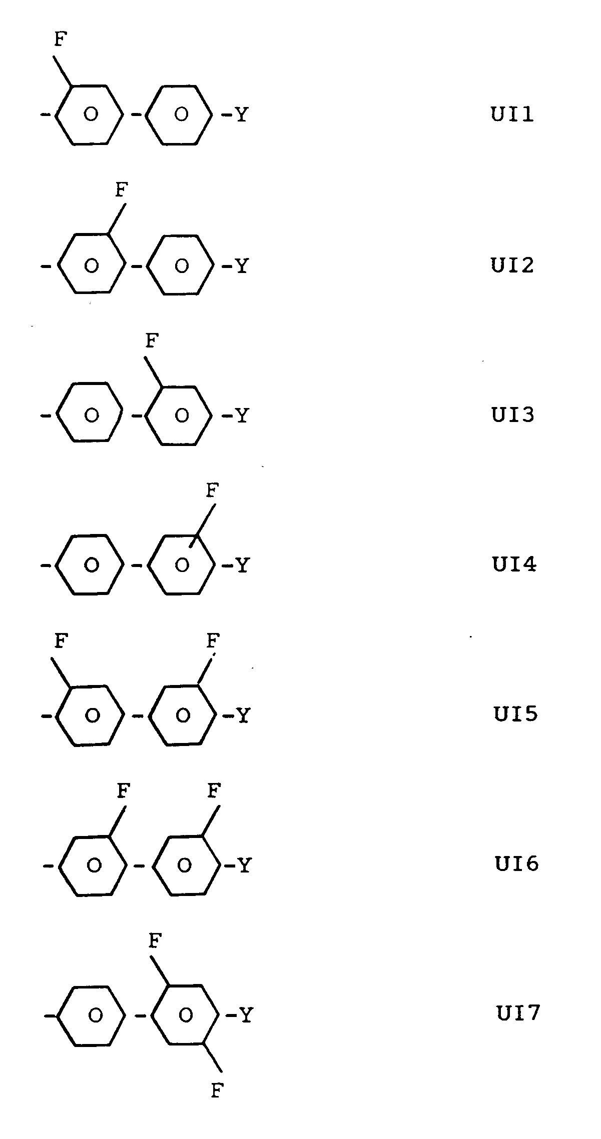 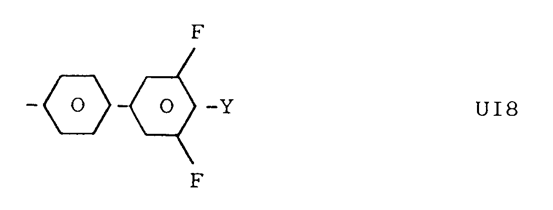
The compounds of the formula I wherein the biphenyl unit UI is a structure element UI1, UI2, UI3, UI4 or UI5 are preferred. The compounds of the formula I wherein Y denotes a -OCF₃ or -OCHF₂ group are particularly preferred.

The fluorinated biphenyl unit UII of the compounds of the formula II 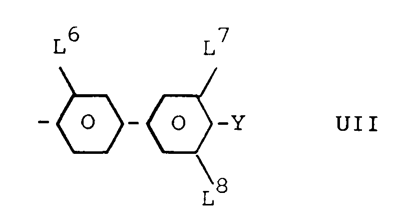
includes the structure elements of UII1 to UII4 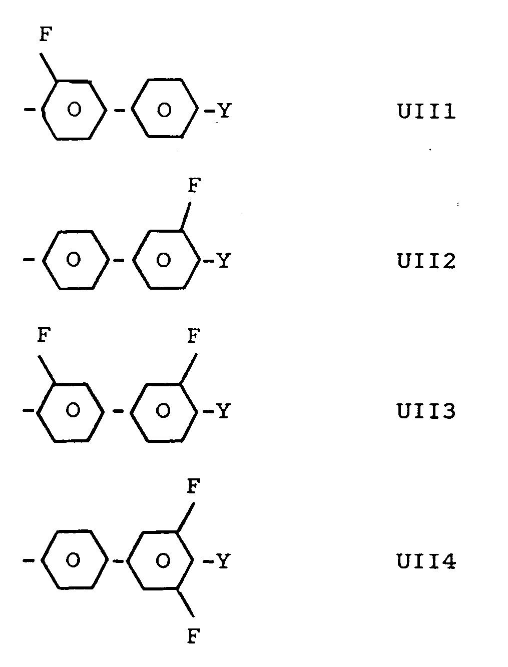
The compounds of the formula II wherein the biphenyl unit UII is a structure element UII1, UII2 or UII4 are preferred. The compounds of the formula II wherein Y denotes -OCF₃ or -OCHF₂ are particularly preferred. The alkylbiphenyl derivatives of the formula III include mono-, di-, tri- and tetra-fluorinated compounds with three or four rings of the formulae III1 to III6. 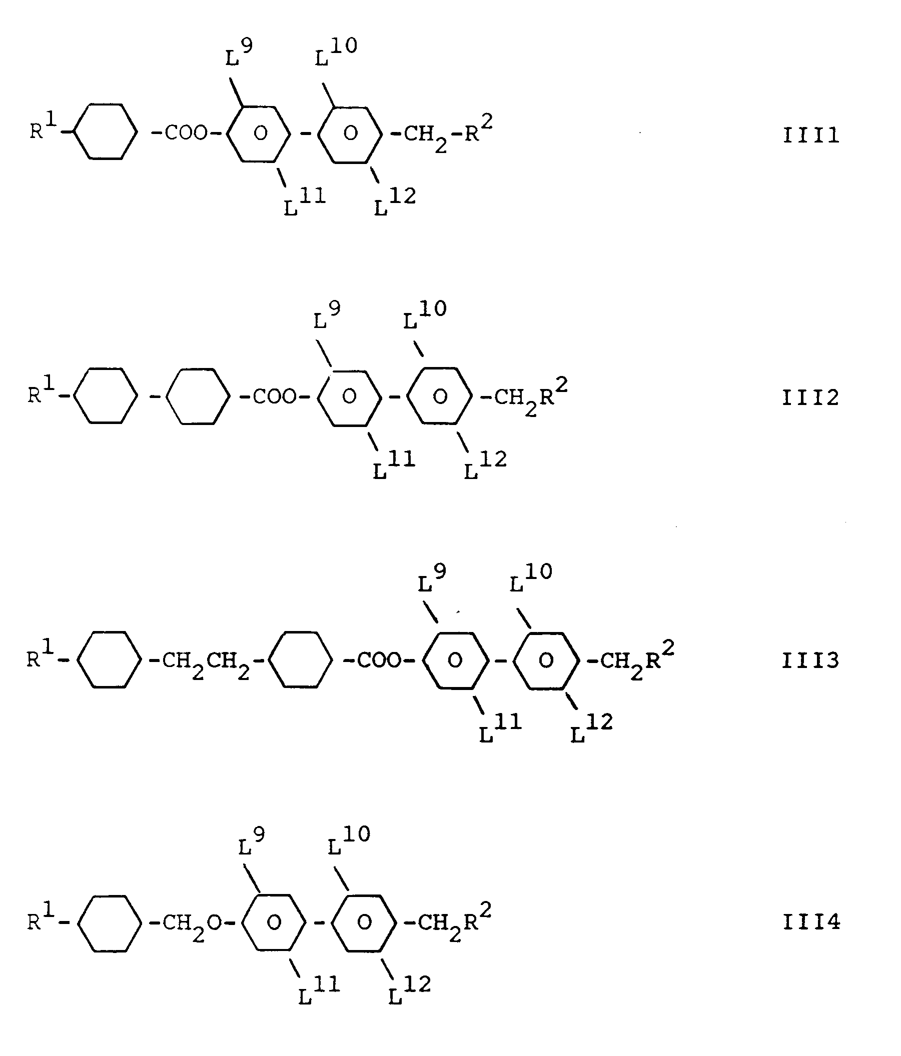 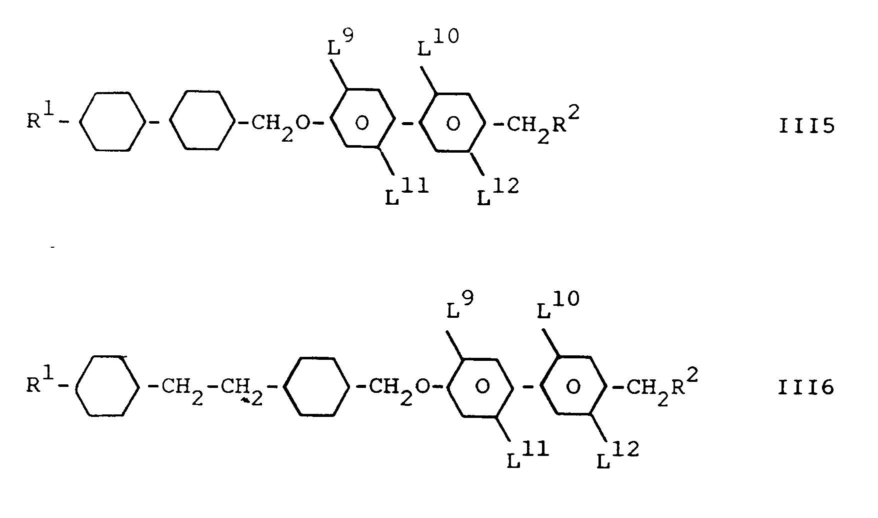
The fluorinated biphenyl UIII 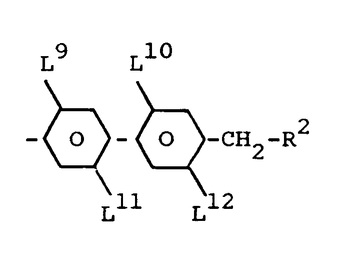
of the compounds of the formula III includes the structure elements UIII1 to UIII 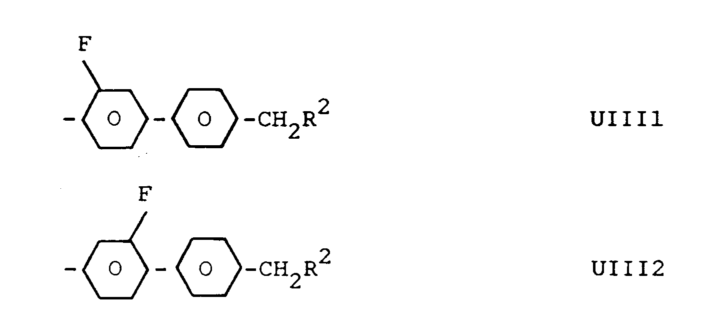 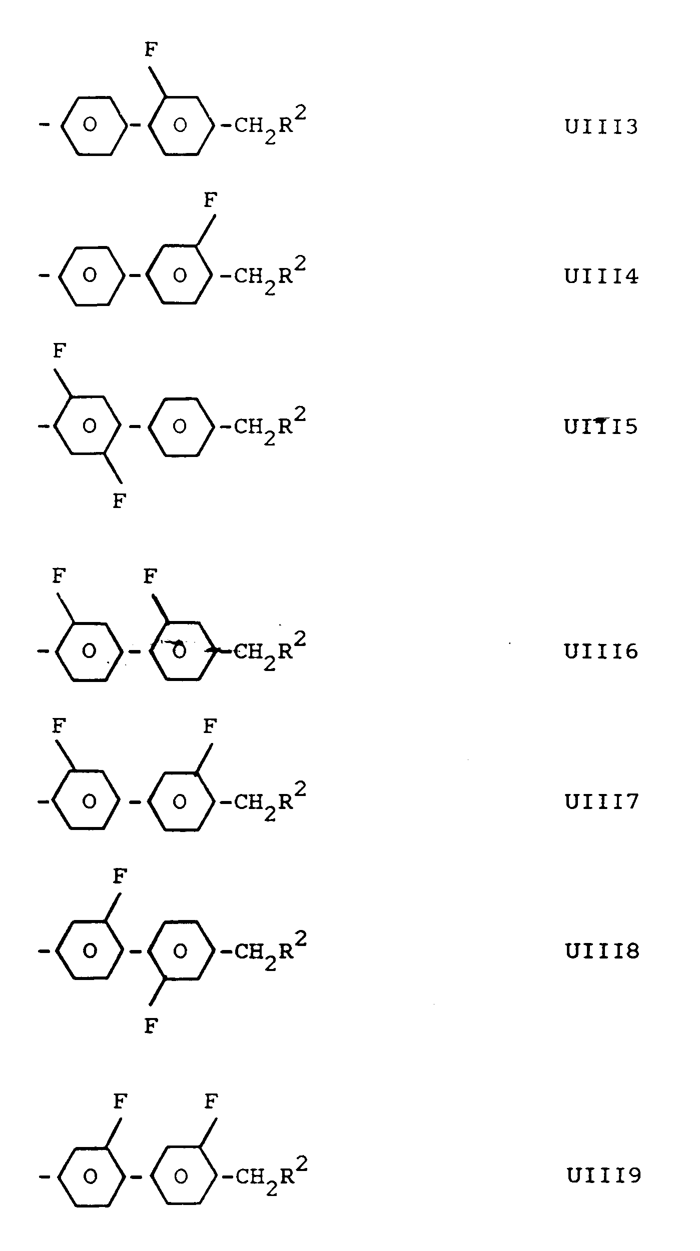 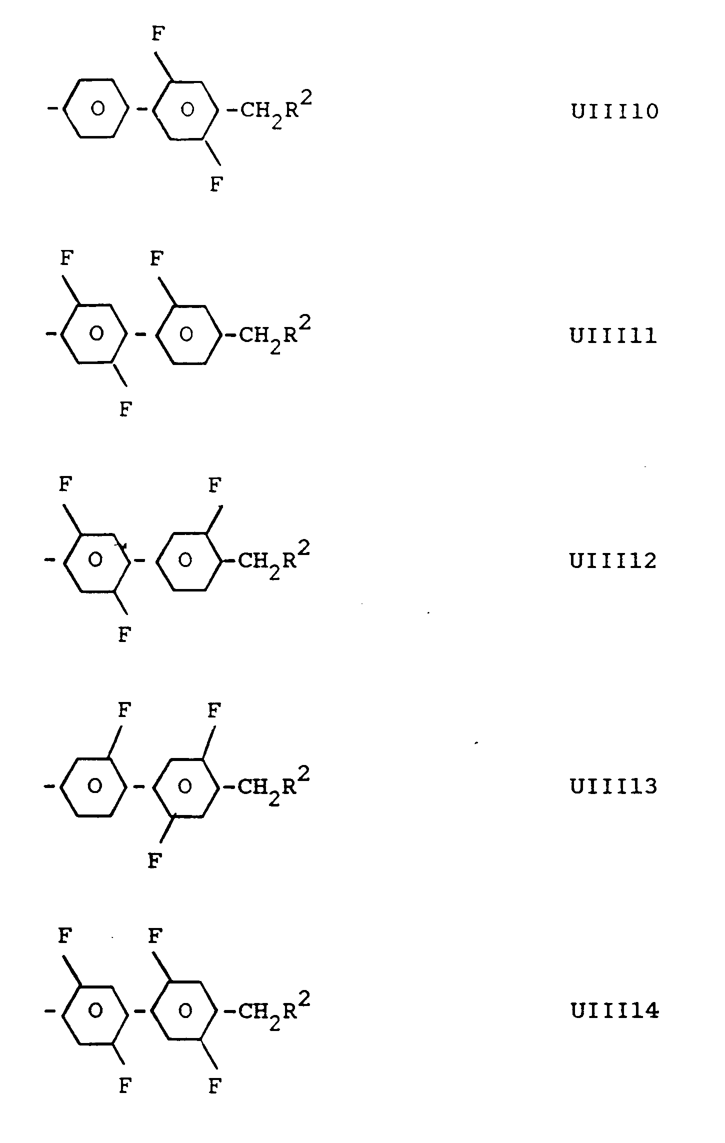
The compounds with three rings (formula I1) are particularly preferred.

Monofluorinated compounds, however, are expecially preferred because they generally exhibit a comperatively low viscosity.

R¹ and/or R² are independently from each other alkyl, alkoxy, oxaalkyl, thioalkyl, alkoxycarbonyl, alkanoyloxy or alkenyl and both can exhibit a straight-chain or branched structure.

Alkyl or alkoxy preferably are straight-chain and have 2, 3, 4, 5, 6 or 7 C atoms. Accordingly they are preferably ethyl, propyl, butyl, pentyl, hexyl, heptyl, ethoxy, propoxy, butoxy, pentoxy, hexoxy or heptoxy, also methyl, octyl, nonyl, decyl, undecyl, dodecyl, tridecyl, tetradecyl, pentadecyl, methoxy, octoxy, nonoxy, decoxy, undecoxy, dodecoxy, tridecoxy or tetradecoxy.

Oxaalkyl is preferably straight-chain 2-oxapropyl (= methoxymethyl), 2-(= ethoxymethyl) or 3-oxybutyl (= 2-methoxyethyl), 2-, 3- or 4-oxapentyl, 2-, 3-, 4- or 5-oxahexyl, 2-, 3-, 4- 5- or 6-oxaheptyl, 2-, 3-, 4-, 5-, 6- or 7-oxaoctyl, 2-, 3-, 4-, 5-, 6-, 7- or 8-oxanonyl or 2-, 3-, 4-, 5-, 6-, 7-, 8-, or 9-oxadecyl.

Alkenyl is preferably straight-chain and has 2 or 10 C atoms. It is accordingly, in particular, vinyl, prop-1- or prop-2-enyl, but-1-, -2- or -3-enyl, pent-1-, -2-, -3- or -4-enyl, hex-1-, -2-, -3-, -4- or -5-enyl, hept-1-, -2-, -3-, -4-, -5- or -6-enyl, oct-1-, -2-, -3-, -4-, -5- -6- or -7-enyl, non-1-, -2-, -3-, -4-, -5-, -6-, -7- or -8-enyl or dec-1-, -2-, -3-, -4-, -5-, -6-, -7-, -8- or -9-enyl.

Compounds of the formula I containing a branched terminal group can occasionally be of importance because of an improved solubility in the customary liquid crystal base materials. Compounds of the formulae I with a branched terminal group R¹ can be used as chiral doping substances if they are optically active. Smectic compounds of this type are suitable as components of ferro-electric materials.

Branched groups of this type as a rule contain not more than one chain branching. Preferred branched radicals are isopropyl, 2-butyl (= 1-methylpropyl), isobutyl (= 2-methylpropyl, 2-methylbutyl, isopentyl, (= 3-methylbutyl), 2-methylpentyl, 2-ethylhexyl, 2-propylpentyl, 2-octyl, isopropoxy, 2-methylpropoxy, 2-methylbutoxy, 3-methylbutoxy, 2-methylpentoxy, 3-methylpentoxy, 2-ethylhexoxy, 2-methylhexoxy, 1-methylhexoxy, 1-methylheptoxy (= 2-octyloxy), 2-oxa-3-methylbutyl, 3-oxa-4-methylpentyl, 4-methylhexyl, 2-nonyl, 2-decyl, 2-dodecyl, 6-methyloctoxy, 6-methyloctanoyloxy, 4-methylheptyloxycarbonyl, 2-methylbutyryloxy, 3-methylvaleryloxy, 4-methylhexanoyloxy, 2-methyl-3-oxapentyl and 2-methyl-3-oxahexyl.

In the case of compounds with a branched terminal group, formulae I include both the optical antipodes and racemates as well as mixtures thereof.

Particularly preferred compounds of the formula I are those of the formulae Ia to Ie: 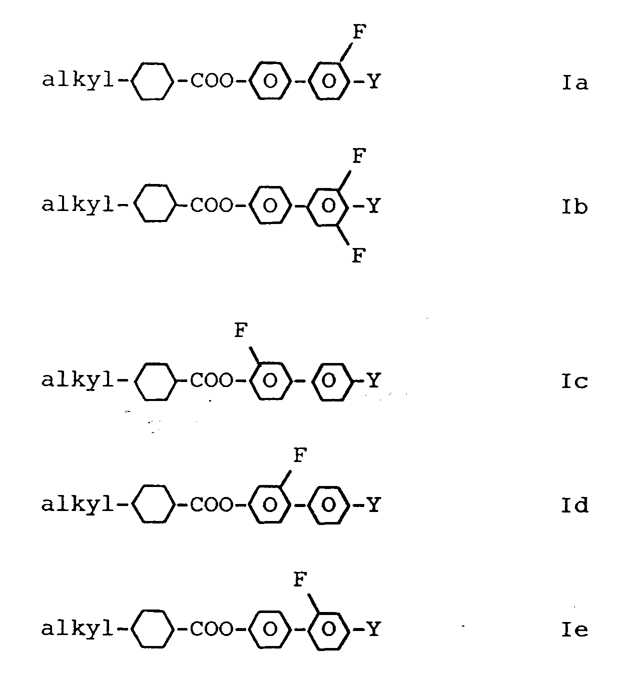
wherein alkyl denotes an alkyl residue with up to 16 C atoms. 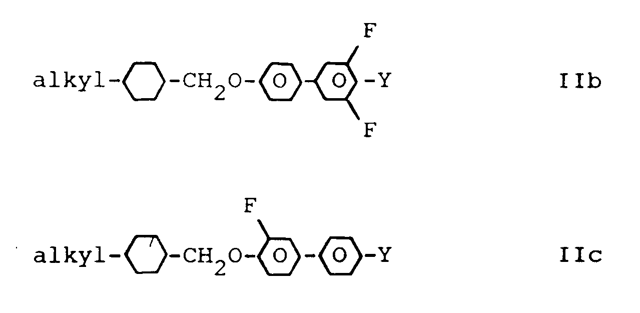
Particularly preferred compounds of the formula III are those of the formulae IIIa to IIIj 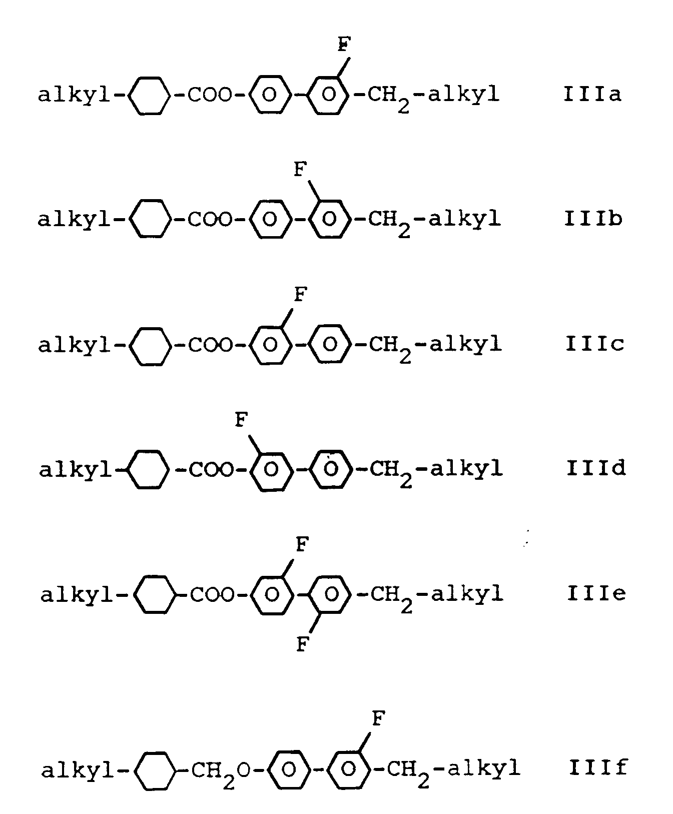
Of the compounds of the formula I and sub-formulae thereof, those in which at least one of the radicals contained therein has one of the preferred meanings given are preferred.

The compounds of the formulae I are prepared by methods which are known per se, such as are described in the literature (for example in the standard works, such as Houben-Weyl, Methoden der Organischen Chemie [Methods of Organic Chemistry], Georg Thieme Verlag, Stuttgart), and in particular under reaction conditions which are known and suitable for the reactions mentioned. Variants which are known per se and are not mentioned in more detail here can also be used in this connection.

If desired, the starting materials can also be formed in situ, such that they are not isolated from the reaction mixture but are immediately reacted further to give the compounds of the formulae I.

Compounds of the formula I can be obtained for example via the esterification of carboxylic acids

R¹-Q-COOH IVa

with biphenols of formulae UI' 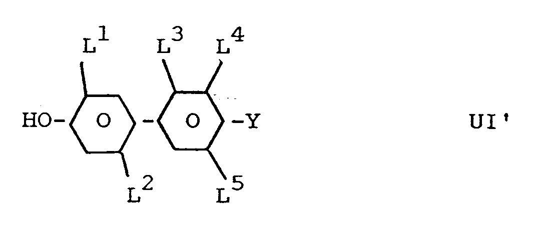
the meaning of R¹, Y, Q and L¹ through L⁵ being indicated above. Other methods of making compounds of formula I are apparent to those skilled in the art.

The biphenols of the formula UI' can be prepared, for example, by coupling p-benzyloxyphenyl boronic acids with, p-bromobenzonitrile, p-bromo-trifluoromethoxybenzene, p-bromo-difluoromethoxybenzene or p-bromo-trifluoromethylbenzene or by coupling the p-trifluoromethoxy-, p-difluoromethoxy-, trifluoromethyl- or-cyanophenyl boronic acids with p-bromobenzyloxybenzene both reactants being appropriately fluorinated and then cleaving of the benzyloxy group hydrogenatically.

In addition to one or more compounds of formula I the liquid crystal compositions according to the invention preferably contain 2-40 components and in particular 4-30 components. Liquid crystal compositions being composed of one or more compounds of formula I and 7-25 other components are especially preferred.

These additional components are preferably chosen from the nematic or nematogenic (monotropic or isotropic) substances; in particular from the classes of azoxybenzenes, benzylideneanilines, biphenyls, terphenyls, phenyl or cyclohexyl benzoates, phenyl or cyclohexyl cyclohexanecarboxylates, phenyl or cyclohexyl cyclohexylbenzoates, phenyl or cyclohexyl cyclohexylcyclohexanecarboxylates, cyclohexylphenylbenzoates, cyclohexylphenyl cyclohexanecarboxylates, cyclohexylphenyl cyclohexylcyclohexanecarboxylates, phenylcyclohexanes, cyclohexylbiphenyls, phenylcyclohexylcyclohexanes, cyclohexylcyclohexanes, cyclohexylcyclohexenes, cyclohexylcyclohexylcyclohexene, 1,4-bis-cyclohexylbenzenes, 4,4'-bis-cyclohexylbiphenyls, phenyl- or cyclohexylpyrimidines, phenyl- or cyclohexylpyridines, phenyl- or cyclohexyldioxanes, phenyl- or cyclohexyl-1,3-dithianes, 1,2-diphenylethanes, 1,2-dicyclohexylethanes, 1-phenyl-2-cyclohexylethanes, 1-cyclohexyl-2-(4-phenyl-cyclohexyl)ethanes, 1-cyclohexyl-2-biphenylethanes, 1-phenyl-2-cyclohexyl-phenylethanes, optionally halogenated stilbenes, benzyl phenyl ethers, tolanes and substituted cinnamic acids.

The 1,4-phenylene groups of these compounds may be fluorinated.

The most important compounds which are possible constituents of liquid crystal compositions according to the invention can be characterized by the formulae 1, 2, 3, 4 and 5:

R′-L-U-R˝ 1

R′-L-COO-U-R˝ 2

R′-L-OOC-U-R˝ 3

R′-L-CH₂CH₂-U-R˝ 4

R′-L-C≡C-U-R˝ 5

In the formulae 1, 2, 3, 4 and 5 L and U may be equal or different from each other. L and U independently from each other denote a bivalent residue selected from the group consisting of -Phe-, -Cyc-, -Phe-Phe-, -Phe-Cyc-, -Cyc-Cyc-, -Pyr-, -Dio-, -G-Phe-, -G-Cyc- and their mirror images; in this compilation of residues Phe denotes unsubstituted or fluorinated 1,4-phenylen, Cyc trans- 1,4-cyclohexylene or 1,4-cyclohexenylen, Pyr pyrimidine-2,5-diyl or pyridine-2,5-diyl, Dio 1,3-dioxane-2,5-diyl and G 2-(trans-1,4-cyclohexyl)-ethyl, pyrimidine-2,5-diyl, pyridine-2,5-diyl or 1,3-dioxane-2,5-diyl.

One of the residues L and U is preferably Cyc, Phe or Pyr. U preferably denotes Cyc, Phe or Phe-Cyc. The liquid crystal media according to the invention preferably contain one or more components selected from the compounds of formulae 1, 2, 3, 4 and 5 with L and U meaning Cyc, Phe and Pyr, said liquid crystal media further containing at the same time one ore more components selected from the compounds of formulae 1, 2, 3, 4 and 5 with one of the residues L and U denoting Cyc, Phe and Pyr and the other residue being selected from the group consisting of -Phe-Phe-, -Phe-Cyc-, -Cyc-Cyc-, -G-Cyc-, said liquid crystal media containing in addition to this optionally one or more components selected from the compounds of formulae 1, 2, 3, 4 and 5 with L and U being selected from the group consisting of -Phe-Cyc-, -Cyc-Cyc-, -G-Phe- and -G-Cyc.

In a preferred subgroup of the compounds of formulae 1, 2, 3, 4 and 5 (subgroup 1) R′ and R˝ are independently from each other alkyl, alkenyl, alkoxy, alkenoxy with up to 8 carbon atoms. R′ and R˝ differ from one another in most of these compounds, one of the residues usually being alkyl or alkenyl. In another preferred subgroup of the compounds of formulae 1, 2, 3, 4 and 5 (subgroup 2) R˝ denotes -CN, -CF₃, -F, -Cl or -NCS while R′ has the meaning indicated in subgroup 1 and is preferably alkyl or alkenyl. Other variants of the envisaged substituents in the compounds of formulae 1, 2, 3, 4 and 5 are also customary. Many such substances are commercially available. All these substances are obtainable by methods which are known from the literature or by analogous methods.

The liquid crystal compositions according to the invention preferably contain in addition to components selected from subgroup 1 also components of subgroup 2, the percentage of these components being as follows:
subgroup 1: 20 to 90 %, in particular 30 to 90 %
subgroup 2: 10 to 50 %, in particular 10 to 50 %
In these liquid crystal media the percentages of the compounds according to the invention and the compounds of sub-group 1 and 2 may add up to give 100 %.

The compositions according to the invention preferably contain 1 to 40 %, in particular 5 to 30 % of the compounds according to the invention. Media containing more than 40 %, in particular 45 to 90 % of the compounds according to the invention are further preferred. The media contain preferably 3, 4 or 5 compounds according to the invention.

The compositions according to the invention are prepared in a manner which is customary per se. As a rule, the components are dissolved in one another, advantageously at elevated temperature. The liquid crystal compositions according to the invention can be modified by suitable additives so that they can be used in all the types of liquid crystal display devices.

Such additives are known to the expert and are described in detail in the literature (H. Kelker/R. Hatz, Handbook of Liquid Crystals, Verlag Chemie, Weinheim, 1980). For example, it is possible to add pleochroic dyestuffs to prepare colored guest-host systems or substances for modifying the dielectric anisotropy, the viscosity and/or the orientation of the nematic phases.

Preferably the compositions according to the invention are used in twisted nematic cells. Twisted nematic cells includes all types of displays, which are based on twisted nematic effect in particular the TN-LCD, the supertwisted STN- and OMI-LCDs, cells which are addressed by a thin film transistor (TFT-LCD) and the SBE-cells.
preferably 50 to 95 %, of the base mixture. Suitable further components with a comparatively low positive or negative dielectric anisotropy are compounds of the formulae 1 to 6. 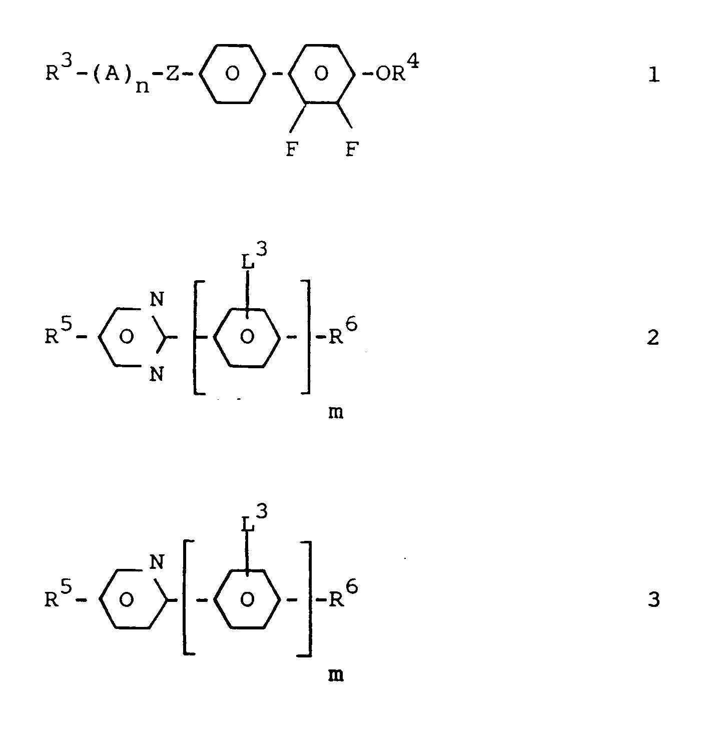 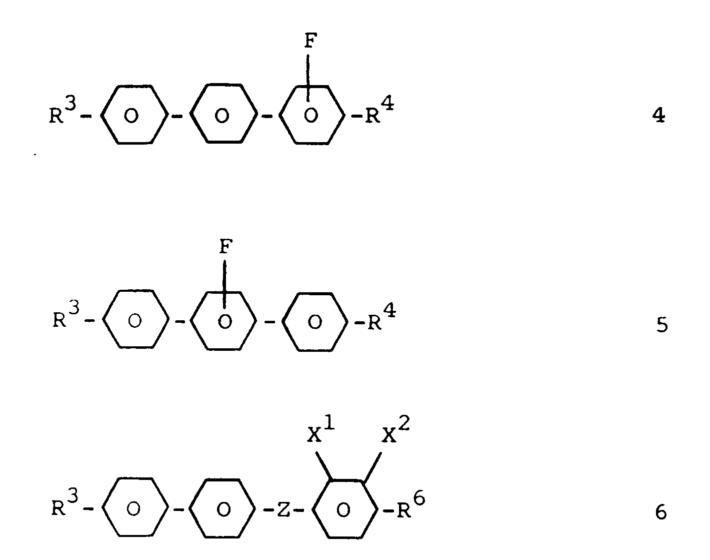
wherein R³ and R⁶ are each alkyl or alkoxy with 5 to 15 C atoms,
Z is -CO-O- or a single bond,
X¹, X² and L³ is hydrogen or fluorine,
R⁴ and R⁵ are each alkyl with 5 to 15 C atoms, m is 1 or 2, n is 0 or 1 and
A is 1,4-cyclohexylene or 1,4-bicyclo[2.2.2]octylene.

The chiral tilted smectic phases according to the invention contain preferably at least three and in particular at least five compounds of the formula III. Particularly preferred chirally tilted smectic liquid crystal phases according to the invention are those in which the achiral base mixture contains, in addition to compounds of the formula III, at least one other component with a negative or comparatively low positive dielectric anisotropy. This/these other component(s) of the chiral base mixture can make up 1 to 50 %, preferably 10 to 25 %, of the base mixture. Suitable further components with a comparatively low positive or negative dielectric anisotropy are compounds of the formula IV, which include the compounds of the part formula IVa to IVi: 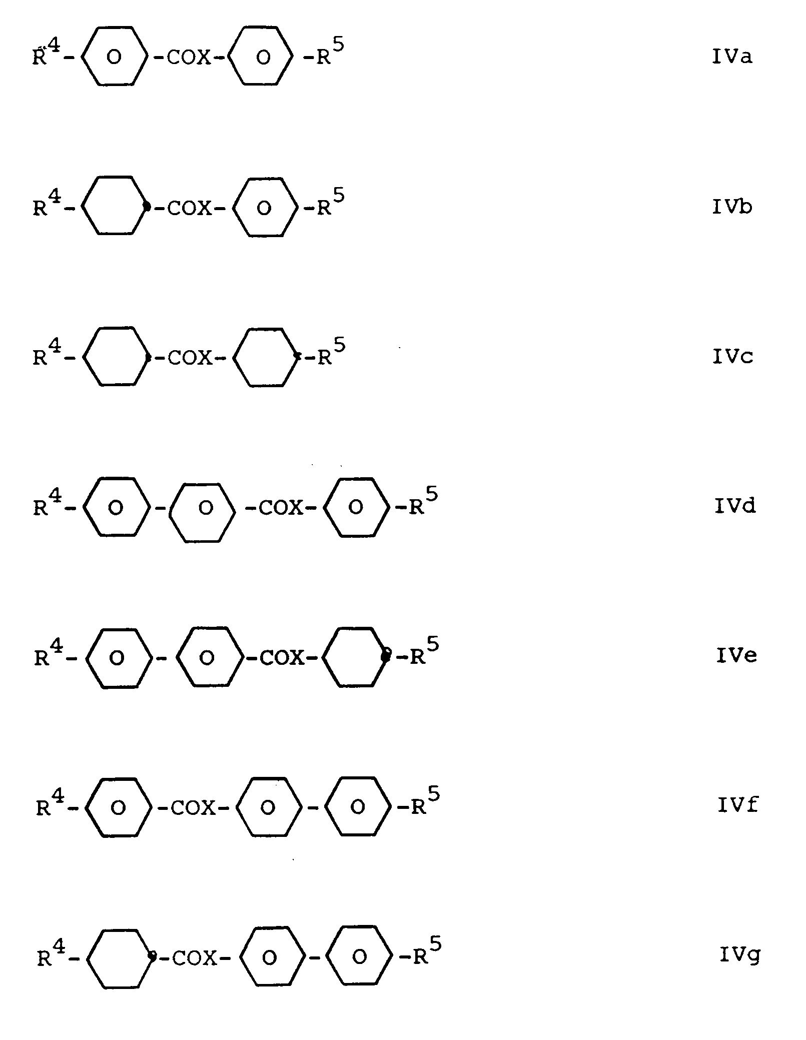 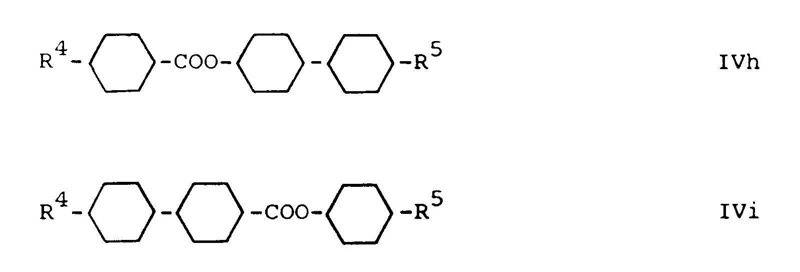
R⁴ and R⁵ are in each case preferably straight-chain alkyl, alkoxy, alkanoyloxy or alkoxycarbonyl with in each case 3 to 12 C atoms. X is O or S, preferably O. In the compounds of the formulae IVa, IVb, IVd, IVe, IVf and IVg, it is also possible for a 1,4-phenylene group to be laterally substituted by halogen or CN, particularly preferably by fluorine.

Particularly preferred compounds are those of the part formulae IVa, IVb, IVd and IVf, wherein R⁴ and R⁵ are each straight-chain alkyl or alkoxy with in each case 5 to 10 C atoms.

Particularly preferred chiral dopants are those of formula VI 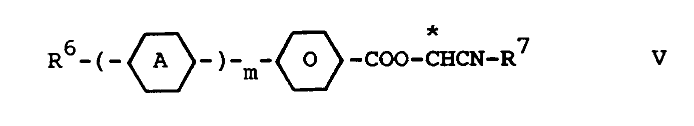
wherein R⁶ is alkyl or alkoxy with 5 to 15 C atoms. R⁷ is alkyl with 1 to 8 C atoms, m is 1 or 2, and 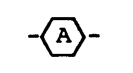
is 1,4-phenylene optionally substituted by fluorine, pyridine-2,5-diyl, pyrimidine-2,5-diyl or trans-1,4-cyclohexylene.

The following examples are to be construed as merely illustrative and not limitative. m.p. = melting point, c.p. = clearing point. In the foregoing and in the following all parts and percentages are by weight and the temperatures are set forth in degrees Celsius. "Customary work-up" means that water is added, the mixture is extracted with methylene chloride, the organic phase is seperated off, dried and evaporated, and the product is purified by crystallization and/or chromatography.

Further are:
C: crystalline-solid state, S: smectic phase (the index denoting the typ of smectic phase), N: nematic phase, Ch: cholesteric phase, I: isotropic phase. The number being embraced by 2 of these symbols denotes the temperature of phase change.

### Example 1

STEP 1 Preparation of 4-bromo-2-fluorobenzyloxybenzene
   A mixture of 4-bromo-2-fluorophenol (0.419 mol), benzylbromide (0.45 mol), potassium carbonate (110 g) and butanone (500 ml) is heated under reflux and stirred for 16 hrs. After cooling, the reaction mixture is filtered and the filtrate evaporated off, distillation under reduced pressure affords the required pure product.
STEP 2 Preparation of 3-fluoro-4-benzyloxyphenylboronic acid
   The product from Step 1 (0.31 mol) is dissolved in dry tetrahydrofuran (250 ml); a small portion of this solution is added to a mixture of tetrahydrofuran and magnesium turnings (8.5 g) and the reaction initiated using iodine and gentle warming. Further bromo-compound solution is added at such a rate so as to maintain a gentle reflux. When all the bromo-compound has been added, the reaction mixture is refluxed for 1 h and then cooled, a further 120 ml of tetrahydrofuran is added. Under a nitrogen atmosphere, the Grignard reagent is slowly added to triisopropylborate (123 g) in tetrahydrofuran (10 ml) at -70 °C. After the addition, the mixture is allowed to warm to 20 °C. 10 % hydrochloric acid solution (500 ml) is added and the organic layer separated, washed with brine and evaporated to give an oil which is subjected to a vacuum of 1 mm Hg at 20 °C for 1 h. The crude product is used in the next step.
STEP 3 Preparation of 4′-benzyloxy-3′-fluoro-4-cyanobiphenyl
   A mixture of the boronic acid from STEP 2
   (0.098 mol), 4-bromobenzonitrile (0.081 mol), tetrakis triphenylphosphine palladium (0.5 g), 2M sodium carbonate (100 ml) methylated spirits (60 ml) and toluene (200 ml) is refluxed and stirred under nitrogen for 16 h. The cooled mixture is poured into water and extracted with dichloromethane, washed with water, dried and evaporated to give a pale yellow solid. After being chromatographed on 150 g of alumina, the purified product is crystallized from cyclohexane.
STEP 4 Preparation of 3′-fluoro-4-cyanobiphenyl-4′-ol
   The product from STEP 3 (0.57 mol) is dissolved in ethyl acetate (200 ml) and hydrogenated over Pd/C (1 g) until no further hydrogen is taken up. The mixture is then filtered, evaporated to dryness and crystallized from methanol.
STEP 5 Preparation of 3′-fluoro-4-cyanobiphenyl-4′-yl-(trans-4-propylcyclohexane carboxylate)
   The product from STEP 4 (0.0067 mol) is dissolved in cold dry dichloromethane (20 ml) and triethylamine (1 ml). Trans-4-propylcyclohexanecarboxyl chloride (0.0073 mol) is added and the mixture stirred at 20 °C for 16 h. After the mixture has been washed with dilute hydrochloric acid and water, it is dried and evaporated to give a low melting solid. The crude product is chromatographed on silica and finally crystallized from methanol/ethyl acetate to give a white solid, C 66 N 216 I.

Analogously are obtained
3′-fluoro-4-cyanobiphenyl-4′-yl-(trans-4-ethylcyclohexanecarboxylate)
3′-fluoro-4-cyanobiphenyl-4′-yl-(trans-4-butylcyclohexanecarboxylate)
3′-fluoro-4-cyanobiphenyl-4′-yl-(trans-4-pentylcyclohexanecarboxylate)
3′-fluoro-4-cyanobiphenyl-4′-yl-(trans-4-hexylcyclohexanecarboxylate)
3′-fluoro-4-cyanobiphenyl-4′-yl-(trans-4-heptylcyclohexanecarboxylate)
3′-fluoro-4-cyanobiphenyl-4′-yl-(trans-4-octylcyclohexanecarboxylate)
3′-fluoro-4-cyanobiphenyl-4′-yl-(trans-4-nonylcyclohexanecarboxylate)
3′-fluoro-4-cyanobiphenyl-4′-yl-(trans-4-decylcyclohexanecarboxylate)
3′-fluoro-4-cyanobiphenyl-4′-yl-(trans-4-dodecylcyclohexanecarboxylate)
2′-fluoro-4-cyanobiphenyl-4′-yl-(trans-4-ethylcyclohexanecarboxylate)
2′-fluoro-4-cyanobiphenyl-4′-yl-(trans-4-propylcyclohexanecarboxylate), C 74.5 N 208 I
2′-fluoro-4-cyanobiphenyl-4′-yl-(trans-4-butylcyclohexanecarboxylate)
2′-fluoro-4-cyanobiphenyl-4′-yl-(trans-4-pentylcyclohexanecarboxylate)
2′-fluoro-4-cyanobiphenyl-4′-yl-(trans-4-hexylcyclohexanecarboxylate)
2′-fluoro-4-cyanobiphenyl-4′-yl-(trans-4-heptylcyclohexanecarboxylate)
2′-fluoro-4-cyanobiphenyl-4′-yl-(trans-4-octylcyclohexanecarboxylate)
2′-fluoro-4-cyanobiphenyl-4′-yl-(trans-4-nonylcyclohexanecarboxylate)
2′-fluoro-4-cyanobiphenyl-4′-yl-(trans-4-decylcyclohexanecarboxylate)
2′-fluoro-4-cyanobiphenyl-4′-yl-(trans-4-dodecylcyclohexanecarboxylate)
3-fluoro-4-cyanobiphenyl-4′-yl-(trans-4-ethylcyclohexanecarboxylate)
3-fluoro-4-cyanobiphenyl-4′-yl-(trans-4-propylcyclohexanecarboxylate)
3-fluoro-4-cyanobiphenyl-4′-yl-(trans-4-butylcyclohexanecarboxylate)
3-fluoro-4-cyanobiphenyl-4′-yl-(trans-4-pentylcyclohexanecarboxylate)
3-fluoro4-cyanobiphenyl-4′-yl-(trans-4-hexylcyclohexanecarboxylate)
3-fluoro-4-cyanobiphenyl-4′-yl-(trans-4-heptylcyclohexanecarboxylate)
3-fluoro-4-cyanobiphenyl-4′-yl-(trans-4-octylcyclohexanecarboxylate)
3-fluoro-4-cyanobiphenyl-4′-yl-(trans-4-nonylcyclohexanecarboxylate)
3-fluoro-4-cyanobiphenyl-4′-yl-(trans-4-decylcyclohexanecarboxylate)
3-fluoro-4-cyanobiphenyl-4′-yl-(trans-4-dodecylcyclohexanecarboxylate)
3′-fluoro-4-(trifluoromethyl)-biphenyl-4′-yl-(trans-4-ethylcyclohexane carboxylate)
3′-fluoro-4-(trifluoromethyl)-biphenyl-4′-yl-(trans-4-propylcyclohexane carboxylate)
3′-fluoro-4-(trifluoromethyl)-biphenyl-4′-yl-(trans-4-butylcyclohexane carboxylate)
3′-fluoro-4-(trifluoromethyl)-biphenyl-4′-yl-(trans-4-pentylcyclohexane carboxylate)
3′-fluoro-4-(trifluoromethyl)-biphenyl-4′-yl-(trans-4-hexylcyclohexane carboxylate)
3′-fluoro-4-(trifluoromethyl)-biphenyl-4′-yl-(trans-4-heptylcyclohexane carboxylate)
3′-fluoro-4-(trifluoromethyl)-biphenyl-4′-yl-(trans-4-octylcyclohexane carboxylate)
3′-fluoro-4-(trifluoromethyl)-biphenyl-4′-yl-(trans-4-nonylcyclohexane carboxylate)
3′-fluoro-4-(trifluoromethyl)-biphenyl-4′-yl-(trans-4-decylcyclohexane carboxylate)
3′-fluoro-4-(trifluoromethyl)-biphenyl-4′-yl-(trans-4-dodecylcyclohexane carboxylate)
2′-fluoro-4-(trifluoromethyl)-biphenyl-4′-yl-(trans-4-ethylcyclohexane carboxylate)
2′-fluoro-4-(trifluoromethyl)-biphenyl-4′-yl-(trans-4-propylcyclohexane carboxylate)
2′-fluoro-4-(trifluoromethyl)-biphenyl-4′-yl-(trans-4-butylcyclohexane carboxylate)
2′-fluoro-4-(trifluoromethyl)-biphenyl-4′-yl-(trans-4-pentylcyclohexane carboxylate)
2′-fluoro-4-(trifluoromethyl)-biphenyl-4′-yl-(trans-4-hexylcyclohexane carboxylate)
2′-fluoro-4-(trifluoromethyl)-biphenyl-4′-yl-(trans-4-heptylcyclohexane carboxylate)
2′ -fluoro-4-(trifluoromethyl)-biphenyl-4′-yl-(trans-4-octylcyclohexane carboxylate)
2′-fluoro-4-(trifluoromethyl)-biphenyl-4′-yl-(trans-4-nonylcyclohexane carboxylate)
2′-fluoro-4-(trifluoromethyl)-biphenyl-4′-yl-(trans-4-decylcyclohexane carboxylate)
2′-fluoro-4-(trifluoromethyl)-biphenyl-4′-yl-(trans-4-dodecylcyclohexane carboxylate)
2′-fluoro-4-(trifluoromethoxy)-biphenyl-4′-yl-(trans-4-ethylcyclohexane carboxylate)
2′-fluoro-4-(trifluoromethoxy)-biphenyl-4′-yl-(trans-4-propylcyclohexane carboxylate)
2′-fluoro-4-(trifluoromethoxy)-biphenyl-4′-yl-(trans-4-butylcyclohexane carboxylate)
2′-fluoro-4-(trifluoromethoxy)-biphenyl-4′-yl-(trans-4-pentylcyclohexane carboxylate)
2′-fluoro-4-(trifluoromethoxy)-biphenyl-4′-yl-(trans-4-hexylcyclohexane carboxylate)
2′-fluoro-4-(trifluoromethoxy)-biphenyl-4′-yl-(trans-4-heptylcyclohexane carboxylate)
2′-fluoro-4-(trifluoromethoxy)-biphenyl-4′-yl-(trans-4-octylcyclohexane carboxylate)
2′-fluoro-4-(trifluoromethoxy)-biphenyl-4′-yl-(trans-4-nonylcyclohexane carboxylate)
2′-fluoro-4-(trifluoromethoxy)-biphenyl-4′-yl-(trans-4-decylcyclohexane carboxylate)
2′-fluoro-4-(trifluoromethoxy)-biphenyl-4′-yl-(trans-4-dodecylcyclohexane carboxylate)
3′-fluoro-4-(trifluoromethoxy)-biphenyl-4′-yl-(trans-4-ethylcyclohexane carboxylate)
3′-fluoro-4-(trifluoromethoxy)-biphenyl-4′-yl-(trans-4-propylcyclohexane carboxylate)
3′-fluoro-4-(trifluoromethoxy)-biphenyl-4′-yl-(trans-4-butylcyclohexane carboxylate)
3′-fluoro-4-(trifluoromethoxy)-biphenyl-4′-yl-(trans-4-pentylcyclohexane carboxylate)
3′-fluoro-4-(trifluoromethoxy)-biphenyl-4′-yl-(trans-4-hexylcyclohexane carboxylate)
3′-fluoro-4-(trifluoromethoxy)-biphenyl-4′-yl-(trans-4-heptylcyclohexane carboxylate)
3′-fluoro-4-(trifluoromethoxy)-biphenyl-4′-yl-trans-4-octylcyclohexane carboxylate)
3′-fluoro-4-(trifluoromethoxy)-biphenyl-4′-yl-(trans-4-nonylcyclohexane carboxylate)
3′-fluoro-4-(trifluoromethoxy)-biphenyl-4′-yl-(trans-4-decylcyclohexane carboxylate)
3′-fluoro-4-(trifluoromethoxy)-biphenyl-4′-yl-(trans-4-dodecylcyclohexane carboxylate)
3′-fluoro-4-(difluoromethoxy)-biphenyl-4′-yl-(trans-4-ethylcyclohexane carboxylate)
3′-fluoro-4-(difluoromethoxy)-biphenyl-4′-yl-(trans-4-propylcyclohexane carboxylate)
3′-fluoro-4-(difluoromethoxy)-biphenyl-4′-yl-(trans-4-butylcyclohexane carboxylate)
3′-fluoro-4-(difluoromethoxy)-biphenyl-4′-yl-(trans-4-pentylcyclohexane carboxylate)
3′-fluoro-4-(difluoromethoxy)-biphenyl-4′-yl-(trans-4-hexylcyclohexane carboxylate)
3′-fluoro-4-(difluoromethoxy)-biphenyl-4′-yl-(trans-4-heptylcyclohexane carboxylate)
3'-fluoro-4-(difluoromethoxy)-biphenyl-4'-yl-(trans-4-octylcyclohexane carboxylate)
3'-fluoro-4-(difluoromethoxy)-biphenyl-4'-yl-(trans-4-nonylcyclohexane carboxylate)
3'-fluoro-4-(difluoromethoxy)-biphenyl-4'-yl-(trans-4-decylcyclohexane carboxylate)
3'-fluoro-4-(difluoromethoxy)-biphenyl-4'-yl-(trans-4-dodecylcyclohexane carboxylate)
STEP 3 Preparation of 3′-fluoro-4-propylbiphenyl-4′-yl-(trans-4-propylcyclohexanecarboxylate)
   The product from STEP 2 (0.0067 mol) is dissolved in cold dry dichloromethane (20 ml) and triethylamine (1 ml). Trans-4-propylcyclohexanecarboxyl chloride (0.0073 mol) is added and the mixture stirred at 20 °C for 16 h. After the mixture has been washed with dilute hydrochloric acid and water, it is dried and eva porated to give a low melting solid. The crude product is chromatographed on silica and finally crystallized from methanol/ethyl acetate to give a white solid, C 78.9 N 182.3 I, Δn 0.166, Δε -0,92 η (20 °C) 37.7 cSt.

Analogously are obtained
3′-fluoro-4-propylbiphenyl-4′-yl-(trans-4-ethylcyclohexanecarboxylate)
3′-fluoro-4-propylbiphenyl-4′-yl-(trans-4-butylcyclohexanecarboxylate)
3′-fluoro-4-propylbiphenyl-4′-yl-(trans-4-pentylcyclohexanecarboxylate)
3′-fluoro-4-propylbiphenyl-4′-yl-(trans-4-hexylcyclohexanecarboxylate)
3′-fluoro-4-propylbiphenyl-4′-yl-(trans-4-heptylcyclohexanecarboxylate)
3′-fluoro-4-propylbiphenyl-4′-yl-(trans-4-octylcyclohexanecarboxylate)
3′-fluoro-4-propylbiphenyl-4′-yl-(trans-4-nonylcyclohexanecarboxylate)
3′-fluoro-4-propylbiphenyl-4′-yl-(trans-4-decylcyclohexanecarboxylate)
3′-fluoro-4-propylbiphenyl-4′-yl-(trans-4-dodecylcyclohexanecarboxylate)
2′-fluoro-4-propylbiphenyl-4′-yl-(trans-4-ethylcyclohexanecarboxylate)
2′-fluoro-4-propylbiphenyl-4′-yl-(trans-4-butylcyclohexanecarboxylate), C 67 N 161 I
2′-fluoro-4-propylbiphenyl-4′-yl-(trans-4-pentylcyclohexanecarboxylate)
2′-fluoro-4-propylbiphenyl-4′-yl-(trans-4-hexylcyclohexanecarboxylate)
2′-fluoro-4-propylbiphenyl-4′-yl-(trans-4-heptylcyclohexanecarboxylate)
2′-fluoro-4-propylbiphenyl-4′-yl-(trans-4-octylcyclohexanecarboxylate)
2′-fluoro-4-propylbiphenyl-4′-yl-(trans-4-nonylcyclohexanecarboxylate)
2′-fluoro-4-propylbiphenyl-4-yl-(trans-4-decylcyclohexanecarboxylate)
2′-fluoro-4-propylbiphenyl-4′-yl-(trans-4-dodecylcyclohexanecarboxylate)
2′-fluoro-4-heptylbiphenyl-4′-yl-(trans-4-ethylcyclohexanecarboxylate)
2′-fluoro-4-heptylbiphenyl-4′-yl-(trans-4-butylcyclohexanecarboxylate)
2′-fluoro-4-heptylbiphenyl-4′-yl-(trans-4-pentylcyclohexanecarboxylate)
2′-fluoro-4-heptylbiphenyl-4′-yl-(trans-4-hexylcyclohexanecarboxylate)
2′-fluoro-4-heptylbiphenyl-4′-yl-(trans-4-heptylcyclohexanecarboxylate), C 44 S₁ 63 S₂ 94.6 S_{c} 104,3 S_{A} 122.9 N 156 I
2′-fluoro-4-heptylbiphenyl-4′-yl-(trans-4-octylcyclohexanecarboxylate)
2′-fluoro-4-heptylbiphenyl-4′-yl-(trans-4-nonylcyclohexanecarboxylate)
2′-fluoro-4-heptylbiphenyl-4′-yl-(trans-4-decylcyclohexanecarboxylate)
2′-fluoro-4-heptylbiphenyl-4′-yl-(trans-4-dodecylcyclohexanecarboxylate)
3-fluoro-4-heptylbiphenyl-4′-yl-(trans-4-ethylcyclohexanecarboxylate)
3-fluoro-4-heptylbiphenyl-4′-yl-(trans-4-butylcyclohexanecarboxylate)
3-fluoro-4-heptylbiphenyl-4′-yl-(trans-4-pentylcyclohexanecarboxylate)
3-fluoro-4-heptylbiphenyl-4′-yl-(trans-4-hexylcyclohexanecarboxylate)
3-fluoro-4-heptylbiphenyl-4′-yl-(trans-4-heptylcyclohexanecarboxylate), C 41 S_{c} 123.7 S_{A} 158 I
3-fluoro-4-heptylbiphenyl-4′-yl-(trans-4-octylcyclohexanecarboxylate)
3-fluoro-4-heptylbiphenyl-4′-yl-(trans-4-nonylcyclohexanecarboxylate)
3-fluoro-4-heptylbiphenyl-4′-yl-(trans-4-decylcyclohexanecarboxylate)
3-fluoro-4-heptylbiphenyl-4′-yl-(trans-4-dodecylcyclohexanecarboxylate)

### Example 2

STEP 1 Preparation of 4-cyano-3-fluorobiphenyl-4'-ol
   4'-Benzyloxy-4-cyano-3-fluorobiphenyl (0.57 mol obtained by coupling of 4-benzyloxyphenyl boronic acid with 2-fluoro-4-bromobenzonitrile as example 1 STEP 3) is hydrogenated as example 1 STEP 4.
STEP 2 Preparation of 3-fluoro-4-cyanobiphenyl-4'-yl (trans-4-propylcyclohexanecarboxylate)
   The product from STEP 1 is esterified with trans-4-propylcyclohexanecarboxyl chloride as example 1 STEP 5.

Analogously are obtained:
3-fluoro-4-cyanobiphenyl-4'-yl (trans-4-ethylcyclohexanecarboxylate)
3-fluoro-4-cyanobiphenyl-4'-yl (trans-4-butylcyclohexanecarboxylate)
3-fluoro-4-cyanobiphenyl-4'-yl (trans-4-pentylcyclohexanecarboxylate)
3-fluoro-4-cyanobiphenyl-4'-yl (trans-4-hexylcyclohexanecarboxylate)
3-fluoro-4-cyanobiphenyl-4'-yl (trans-4-heptylcyclohexanecarboxylate)
3-fluoro-4-cyanobiphenyl-4'-yl (trans-4-octylcyclohexanecarboxylate)
3-fluoro-4-cyanobiphenyl-4'-yl (trans-4-nonylcyclohexanecarboxylate)
3-fluoro-4-cyanobiphenyl-4′-yl (trans-4-decylcyclohexanecarboxylate)
3-fluoro-4-cyanobiphenyl-4′-yl (trans-4-dodecylcyclohexanecarboxylate)
3-fluoro-4-cyanobiphenyl-4′-yl (trans, trans-4′-ethylbicyclohexylcarboxylate)
3-fluoro-4-cyanobiphenyl-4′-yl (trans, trans-4′-propylbicyclohexylcarboxylate)
3-fluoro-4-cyanobiphenyl-4′-yl (trans, trans-4′-butylbicyclohexylcarboxylate)
3-fluoro-4-cyanobiphenyl-4′-yl (trans, trans-4′-pentylbicyclohexylcarboxylate)
3-fluoro-4-cyanobiphenyl-4′-yl (trans, trans-4′-hexylbicyclohexylcarboxylate)
3-fluoro-4-cyanobiphenyl-4′-yl (trans, trans-4′-heptylbicyclohexylcarboxylate)
3-fluoro-4-cyanobiphenyl-4′-yl (trans, trans-4′-octylbicyclohexylcarboxylate)
3-fluoro-4-cyanobiphenyl-4′-yl (trans, trans-4′-nonylbicyclohexylcarboxylate)
3-fluoro-4-cyanobiphenyl-4′-yl (trans, trans-4′-decylbicyclohexylcarboxylate)
3-fluoro-4-cyanobiphenyl-4′-yl (trans, trans-4′-dodecylbicyclohexylcarboxylate)
3-fluoro-4-propylbiphenyl-4′-yl (trans-4-ethylcyclohexanecarboxylate)
3-fluoro-4-propylbiphenyl-4′-yl (trans-4-propylcyclohexanecarboxylate)
3-fluoro-4-propylbiphenyl-4′-yl (trans-4-butylcyclohexanecarboxylate)
3-fluoro-4-propylbiphenyl-4'-yl (trans-4-pentylcyclohexanecarboxylate)
3-fluoro-4-propylbiphenyl-4'-yl (trans-4-octylcyclohexanecarboxylate)
3-fluoro-4-pentybiphenyl-4'-yl (trans-4-ethylcyclohexanecarboxylate)
3-fluoro-4-pentylbiphenyl-4'-yl (trans-4-propylcyclohexanecarboxylate)
3-fluoro-4-pentylbiphenyl-4'-yl (trans-4-butylcyclohexanecarboxylate)
3-fluoro-4-pentylbiphenyl-4'-yl (trans-4-pentylcyclohexanecarboxylate)
3-fluoro-4-pentylbiphenyl-4'-yl (trans-4-hexylcyclohexanecarboxylate)
3-fluoro-4-pentylbiphenyl-4'-yl (trans-4-heptylcyclohexanecarboxylate)
3-fluoro-4-pentylbiphenyl-4'-yl (trans-4-octylcyclohexanecarboxylate)
3-fluoro-4-pentylbiphenyl-4'-yl (trans-4-nonylcyclohexanecarboxylate)
3-fluoro-4-pentylbiphenyl-4'-yl (trans-4-decylcyclohexanecarboxylate)
3-fluoro-4-pentylbiphenyl-4'-yl (trans-4-dodecylcyclohexanecarboxylate)
trans-4-propylcyclohexylbormomethane (0.1 mol) and dimethylformamide (100 ml) is heated to 120 °C for 15 h. After cooling the inorganic salts are filed and the filtrate is evaporated off. The residue is resolved in dichloromethane, washed with water, dried and evaporated to give a pale yellow solid. After being chromatographed the purified product is crystallized to yield a white solid, C 75 N 115.6 I.

Analogously are obtained:
(2′-fluoro-4-propylbiphenyl-4′-yl)-(trans-4-ethylcyclohexylmethyl)-ether
(2′-fluoro-4-propylbiphenyl-4′-yl)-(trans-4-butylcyclohexylmethyl)-ether
(2′-fluoro-4-propylbiphenyl-4′-yl)-(trans-4-pentylcyclohexylmethyl)-ether
(2′-fluoro-4-propylbiphenyl-4′-yl)-(trans-4-hexylcyclohexylmethyl)-ether
(2′-fluoro-4-propylbiphenyl-4′-yl)-(trans-4-heptylcyclohexylmethyl)-ether
(2′-fluoro-4-propylbiphenyl-4′-yl)-(trans-4-octylcyclohexylmethyl)-ether
(2′-fluoro-4-propylbiphenyl-4′-yl-(trans-4-nonylcyclohexylmethyl)-ether
(2′-fluoro-4-propylbiphenyl-4′-yl)-(trans-4-decylcyclohexylmethyl)-ether
(2′-fluoro-4-propylbiphenyl-4′-yl)-(trans-4-dodecylcyclohexylmethyl)-ether
(2′-fluoro-4-heptylbiphenyl-4′-yl)-(trans-4-ethylcyclohexylmethyl)-ether
(2′-fluoro-4-heptylbiphenyl-4′-yl)-(trans-4-butylcyclohexylmethyl)-ether
(2′-fluoro-4-heptylbiphenyl-4′-yl)-(trans-4-pentylcyclohexylmethyl)-ether
(2′-fluoro-4-heptylbiphenyl-4′-yl)-(trans-4-hexylcyclohexylmethyl)-ether
(2′-fluoro-4-heptylbiphenyl-4′-yl)-(trans-4-heptylcyclohexylmethyl)-ether
(2′-fluoro-4-heptylbiphenyl-4′-yl)-(trans-4-octylcyclohexylmethyl)-ether
(2′-fluoro-4-heptylbiphenyl-4′-yl)-(trans-4-nonylcyclohexylmethyl)-ether
(2′-fluoro-4-heptylbiphenyl-4′-yl)-(trans-4-decylcyclohexylmethyl)-ether
(2′-fluoro-4-heptylbiphenyl-4′-yl)-(trans-4-dodecylcyclohexylmethyl)-ether
(3′-fluoro-4-propylbiphenyl-4′-yl)-(trans-4-ethylcyclohexylmethyl)-ether
(3′-fluoro-4-propylbiphenyl-4′-yl)-(trans-4-propylcyclohexylmethyl)-ether, C 60 N 124.7 I
(3′-fluoro-4-propylbiphenyl-4′-yl)-(trans-4-pentylcyclohexylmethyl)-ether
(3′-fluoro-4-propylbiphenyl-4′-yl)-(trans-4-hexylcyclohexylmethyl)-ether
(3′-fluoro-4-propylbiphenyl-4′-yl)-(trans-4-heptylcyclohexylmethyl)-ether
(3′-fluoro-4-propylbiphenyl-4′-yl)-(trans-4-octylcyclohexylmethyl)-ether
(3′-fluoro-4-propylbiphenyl-4′-yl)-(trans-4-nonylcyclohexylmethyl)-ether

### Example 3

A liquid crystalline medium is obtained containing
21.672 % of 4-(trans-4-propylcyclohexyl)-benzonitrile
32.508 % of 4-(trans-4-pentylcyclohexyl)-benzonitrile
22.575 % of 4-(trans-4-heptylcyclohexyl)-benzonitrile
13.545 % of 4-(trans-4-pentylcyclohexyl)-4'-cyanobiphenyle and
9.700 % of 2'-fluoro-4-cyanobiphenyl-4'-yl-(trans-4-propylcyclohexane carboxylate)
and exhibits N 69 I.

### Example 4

A liquid crystalline medium is obtained containing
28.33 % of 1-(trans-4-propylcyclohexyl)-2-(2'-fluoro-4'-ethylbiphenyl-4-yl)-ethane
28.33 % of 1-(trans-4-propylcyclohexyl)-2-(2'-fluoro-4'-pentylbiphenyl-4-yl)-ethane
28.33 % of 1-(trans-4-pentylcyclohexyl)-2-(2'-fluoro-4'-ethylbiphenyl(4-yl)-ethane
15.00 % of 3'-fluoro-4-propylbiphenyl-4'-yl-(trans-4-propylcyclohexane carboxylate)
and exhibits N 113 I, Δn 0.1527 Δε -0.16 and η (20 °C) 26.5 cSt.

### Example 5

A liquid crystalline medium is obtained containing
21.6 % of 4-(trans-4-propylcyclohexyl)-benzonitrile
32.4 % of 4-(trans-4-pentylcyclohexyl)-benzonitrile
22.5 % of 4-(trans-4-heptylcyclohexyl)-benzonitrile
13.5 % of 4-(trans-4-pentylcyclohexyl)-4'-cyanobiphenyle and
10 % of 2'-fluoro-4-cyanobiphenyl-4'-yl-(trans-4-propylcyclohexanecarboxylate)
and exhibits N 80.3-83.2 I, Δn 0.14462 and η (20 °C) 34.29 cSt.

### Example 6

A liquid crystalline medium is obtained containing
10 % of 4-cyano-3-fluorophenyl 4-ethylbenzoate
9 % of 4-cyano-3-fluorophenyl 4-propylbenzoate
5 % of 4-cyano-3-fluorophenyl 4-pentylbenzoate
11 % of 4-(5-propylpyrimidine-2-yl)-2-fluorobenzonitrile
7.24 % of 4-pentylphenyl 4-methylbenzoate
8.5 % of 4-pentylphenyl 4-propylbenzoate
4.26 % of 4-pentylphenyl 4-pentylbenzoate
7 % of (5-propyldioxane-2-yl)-benzonitrile
5 % of 4-pentyl-3-fluorophenyl (trans,trans-4-pentylbicyclohexyl-4'-yl-carboxylate)
20 % of 2'-fluoro-4-cyanobiphenyl-4'-yl-(trans-4-propylcyclohexylcarboxylate)
and exhibits N 56 I and Δn 0.167. This liquid crystalline medium has a threshold voltage of 0.72 V in a 7 »m thick TN-cell.

## Claims

1. A liquid crystalline composition for the use in a twisted nematic cell being an admixture of a group (A) with one or more components exhibiting a positive dielectric anisotropy (Δε) and a group (B) with one or more components exhibiting a neutral dielectric anisotropy characterized in that
at least one component of group (A) is a compound of the formula I 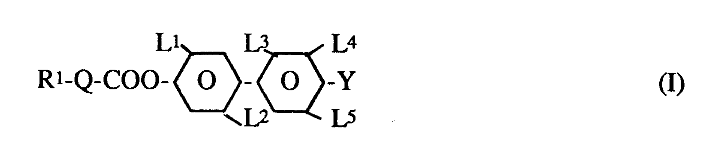 wherein
R¹ is an alkyl or alkenyl residue each with up to 16 C atoms, wherein one or two non-adjacent CH₂-groups of these residues may be replaced by -O-,-S-, -CO-O-, or -O-CO-,
Y is CN, OCF₃, OCHF₂, or CF_{3,}
Q is a group of the formula 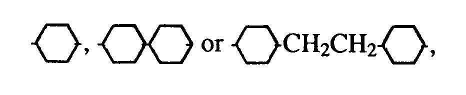 and
one of the lateral ligands L¹ through L⁴ is F, the others are H, and
L⁵ is F or H.

2. A liquid crystalline composition according to claim 1, characterized in that at least one component of group (A) is selected from the compounds of the formulae Ia to Ie 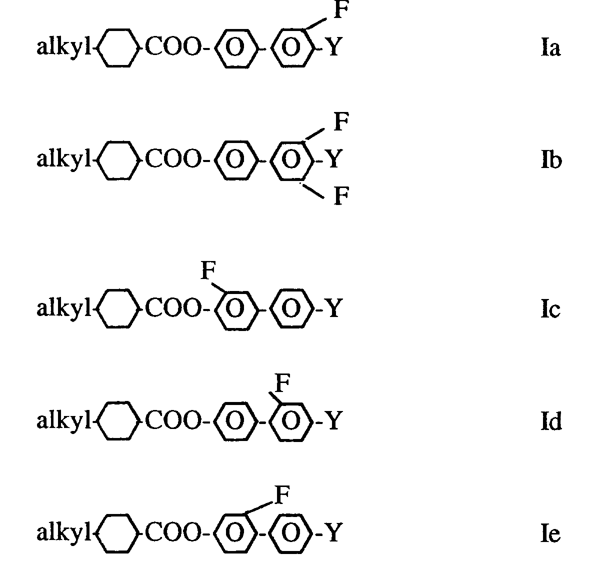 wherein Y has the meaning given and alkyl denotes an alkyl residue with up to 16 C atoms.

3. A liquid crystalline composition according to at least one of the claims 1 or 2 characterized in that the dielectric anisotropy (Δε) of the group A is greater than +6.

4. A liquid crystal composition according to at least one of the claims 1 to 3 characterized in that the dielectric anisotropy (Δε) of the group B is -4 to +3.

5. A twisted nematic cell containing a liquid crystalline composition according to at least one of claims 1 to 4.

6. A cell according to claim 5 characterized in that the twist angle is between 160 and 300°.

7. Compound of the formula I, 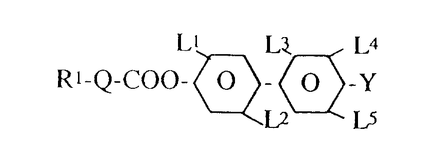 wherein R¹ and Q, L¹ through L⁵ have the meaning given in claim 1, and Y denotes -OCF₂H or -OCF₃.

## Patentansprüche

1. Flüssigkristalline Zusammensetzungen zur Verwendung in einer verdrillt nematischen Zelle, bei denen es sich um ein Gemisch aus einer Gruppe (A) mit einer oder mehreren Komponenten mit positiver dielektrischer Anisotropie (Δε) und einer Gruppe (B) mit einer oder mehreren Komponenten mit neutraler dielektrischer Anisotropie handelt, dadurch gekennzeichnet, daß
mindestens eine Komponente aus Gruppe (A) eine Verbindung der Formel I ist, 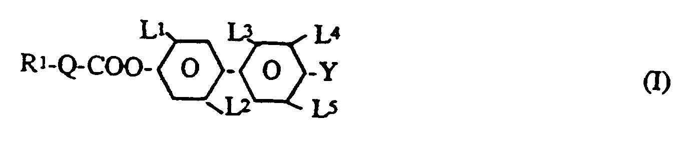 worin
R¹ einen Alkyl- oder Alkenylrest mit jeweils bis zu 16 C-Atomen, worin eine oder zwei nichtbenachbarte CH₂-Gruppen auch durch -O-, -S-, -CO-O- oder -O-CO- ersetzt sein können,
Y CN, OCF₃, OCHF₂ oder CF₃,
Q eine Gruppe der Formel 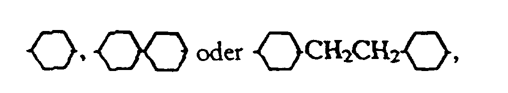 und einer der seitlichen Liganden L¹ bis L⁴ F und die anderen H und
L⁵ F oder H bedeuten.

2. Flüssigkristalline Zusammensetzungen nach Anspruch 1, dadurch gekennzeichnet, daß mindestens eine Komponente aus Gruppe (A) ausgewählt ist aus den Verbindungen der Formeln Ia bis Ie: 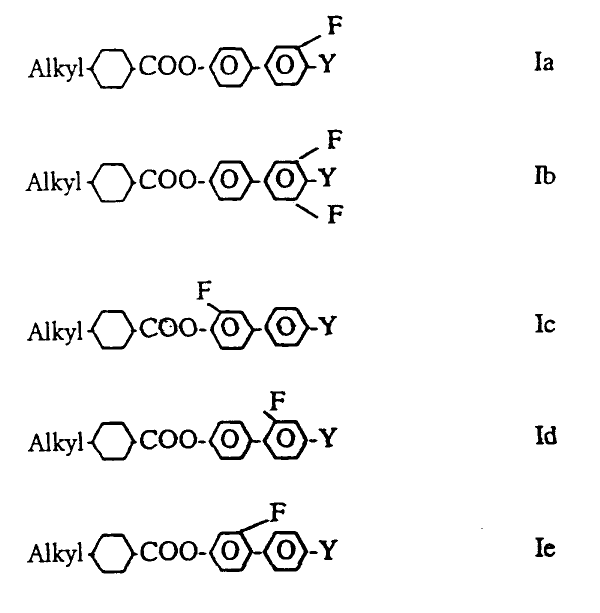 worin Y die angegebene Bedeutung besitzt und Alkyl einen Alkylrest mit bis zu 16 Kohlenstoffatomen bedeutet.

3. Flüssigkristalline Zusammensetzungen nach mindestens einem der Ansprüche 1 oder 2, dadurch gekennzeichnet, daß die dielektrische Anisotropie (Δε) der Gruppe A mehr als +6 beträgt.

4. Flüssigkristalline Zusammensetzungen nach mindestens einem der Ansprüche 1 bis 3, dadurch gekennzeichnet, daß die dielektrische Anisotropie (Δε) der Gruppe B -4 bis +3 beträgt.

5. Verdrillt nematische Zelle, enthaltend eine flüssigkristalline Zusammensetzung nach mindestens einem der Ansprüche 1 bis 4.

6. Zelle nach Anspruch 5, dadurch gekennzeichnet, daS der Verdrillungswinkel zwischen 160 und 300° liegt.

7. Verbindungen der Formel I, 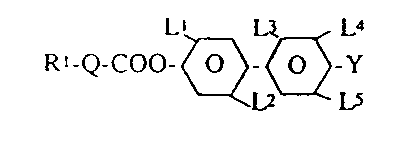 worin R¹, Q und L¹ bis L⁵ die in Anspruch 1 angegebene Bedeutung besitzen und
Y -OCF₂H oder -OCF₃ bedeutet.

## Revendications

1. Composition cristalline liquide utilisable dans une cellule nématique à torsion constituée par un mélange d'un groupe (A) de un ou plusieurs composants présentant une anisotropie diélectrique positive (Δε) et d'un groupe (B) de un ou plusieurs composants présentant une anisotropie diélectrique neutre, caractérisée en ce que
au moins un composant du groupe (A) est un composé de formule I 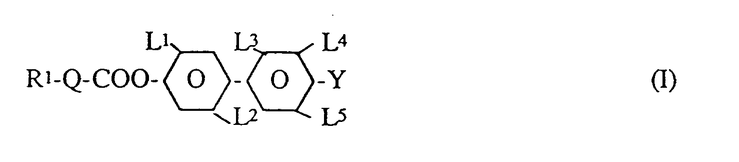 dans laquelle
R¹ est un radical alkyle ou alkényle ayant chacun jusqu'à 16 atomes C, dans lequel un ou deux groupes CH₂- non adjacents de ces radicaux peuvent être remplacés par -O-, -S-, -CO-O-, ou -O-CO-,
Y est CN, OCF₃, OCHF₂, ou CF₃
Q est un groupe de formule 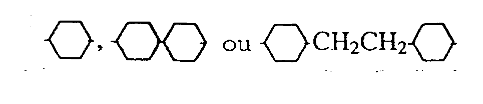 et
un des ligands latéraux L¹ à L⁴ est F, les autres sont H, et
L⁵ est F ou H.

2. Composition cristalline liquide selon la revendication 1, caractérisée en ce que au moins un composant du groupe (A) est choisi parmi les composés des formules Ia à Ie 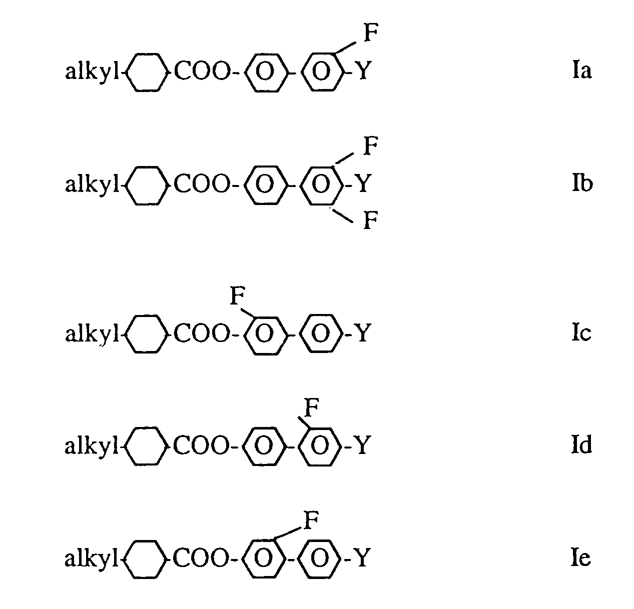 dans lesquelles Y a la signification donnée et alkyl indique un radical alkyle ayant jusqu'à 16 atomes C.

3. Composition cristalline liquide selon au moins une des revendications 1 ou 2, caractérisée en ce que l'anisotropie diélectrique (Δε) du groupe A est supérieure à +6.

4. Composition cristalline liquide selon au moins une des revendications 1 à 3, caractérisée en ce que l'anisotropie diélectrique (Δε) du groupe B est de -4 à +3.

5. Cellule nématique à torsion contenant une composition cristalline liquide conforme à l'une au moins des revendications 1 à 4.

6. Cellule selon la revendication 5, caractérisé en ce que l'angle de torsion est compris entre 160 et 300°C.

7. Composés de formule I, 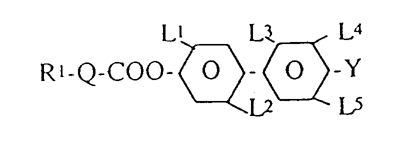 dans laquelle R¹ et Q, L¹ à L⁵ ont la signification donnée dans la revendication 1, et Y représente -OCF₂H ou -OCF₃.
